# EUROPEAN PATENT APPLICATION

(11) **EP 4 745 577 A1**
(43) Date of publication of application: **20.05.2026**
(21) Application number: 24213434.4
(22) Date of filing: 15.11.2024
(51) Int. Cl.: G01N 33/68, G01N 33/74

(54) **A METHOD FOR SCREENING FOR PREECLAMPSIA**

(71) Applicant: University College Dublin, Dublin 4 (IE)
(72) Inventor: Maguire, Patricia, Dublin 4 (IE); O'Gorman, Neil, Dublin 4 (IE); Fouhy, Ella, Dublin 4 (IE)
(74) Representative: FRKelly

(57) **Abstract**

The present invention relates to methods of diagnosing preeclampsia in a subject based on the quantitative level of one or more biomarkers in a sample from the subject. Also disclosed are methods of treating the subject diagnosed with preeclampsia.

## Description

### Field of the invention

The present invention relates to methods of diagnosing preeclampsia in a subject based on the quantitative level of one or more biomarkers in a sample from the subject. Also disclosed are methods of treating the subject diagnosed with preeclampsia.

### Background to the invention

Preeclampsia (PE) (defined as new hypertension presenting after 20 weeks with significant proteinuria or other organ dysfunction) is a serious complication of pregnancy with potentially life-threatening consequences for both mother and baby.

PE is a major public health issue, affecting more than half a million pregnancies in the United States each year and remaining a leading cause of death in the UK and Ireland. Worldwide, an estimated 50,000 women worldwide die annually from PE. A third of babies are born prematurely.

Early screening for PE is imperative to identify subjects at high risk for the disease and those subjects that would benefit from closer monitoring throughout pregnancy. Not only can stratifying subjects at high-risk lead to better and more personalised prenatal care, treatment such as aspirin can also be administered, which has demonstrated efficacy to effectively reduce the risk of preterm PE before 34 weeks and 37 weeks by 62% and 80% respectively, when taken before 16 weeks' gestation.

Currently the most accurate method of first trimester screening method for preeclampsia has a detection rate of approximately 80% with a false positive rate of 10% for developing the disorder prior to 37 weeks gestation. This method involves a complex mix of the maternal history with both biophysical and biochemical markers entered into an algorithm. This method has been difficult to implement clinically due to its cumbersome methodology and consequently has not been widely accepted outside a research setting. There are no other accurate clinically recommended screening tests for PE before this time. Screening often occurs based primarily on maternal factors alone at 11-13 weeks' gestation which can only detect 49% of those at high risk of the disease.

Current biomarkers for PE in early pregnancy are low performing and are not used by themselves, but rather in conjunction with other maternal demographics and other highly specialised biophysical measurements.

Pregnancy management is often reactive rather than proactive. Therefore, by screening in the early weeks of pregnancy, future disease risk can be effectively reduced for severe pregnancy complications that often only become apparent in later stages of pregnancy.

Maternal and foetal outcomes can be improved by their stratification as high risk early in gestation, in keeping with the inverted pyramid of care. Clinicians therefore desperately need better tools to identify high risk women in early pregnancy who would benefit from aspirin prophylaxis to reduce their risk of PE.

Novel biomarkers are therefore urgently needed to identify women at high risk in early pregnancy.

Additionally, more specific biomarkers that can be used on their own without extensive/specialist biophysical measurements can make screening for PE in early pregnancy accessible and routine.

### Summary of the invention

According to a first aspect of the present invention, there is provided a method of diagnosing preeclampsia in a subject, the method comprising the steps of:
(a) determining the quantitative level of one or more biomarkers in a sample from the subject; and
(b) diagnosing preeclampsia in the subject based on the quantitative level of the or each biomarker in the sample;
wherein the or each biomarker is selected from Dickkopf-related protein 1 (Dkk1) and platelet factor 4 (PF4).

Optionally, the biomarker is Dickkopf-related protein 1.

Optionally, the biomarker is platelet factor 4.

Optionally, the biomarker is platelet factor 4 and Dickkopf-related protein 1.

Optionally, the diagnosing step (b) comprises comparing the quantitative level of the or each biomarker in the sample from the subject with the quantitative level of the or each respective biomarkers in a normal sample.

Optionally, the normal sample is a sample from a subject not suffering from preeclampsia.

Optionally, the normal sample is a sample from a healthy subject.

Optionally, a quantitative level of the or each biomarker in the sample from the subject greater than a quantitative level of the or each respective biomarkers in a normal sample is indicative of preeclampsia.

Optionally, a quantitative level of Dickkopf-related protein 1 and/or platelet factor 4 in the sample from the subject greater than a quantitative level of Dickkopf-related protein 1 and/or platelet factor 4 in a normal sample is indicative of preeclampsia.

Optionally, a quantitative level of Dickkopf-related protein 1 in the sample from the subject greater than a quantitative level of Dickkopf-related protein 1 in a normal sample is indicative of preeclampsia.

Optionally, a quantitative level of Dickkopf-related protein 1 in the sample from the subject greater than a quantitative level of Dickkopf-related protein 1 in a normal sample is indicative of term preeclampsia.

Optionally, a quantitative level of platelet factor 4 in the sample from the subject greater than a quantitative level of platelet factor 4 in a normal sample is indicative of preeclampsia.

Optionally, a quantitative level of platelet factor 4 and Dickkopf-related protein 1 in the sample from the subject greater than a quantitative level of platelet factor 4 and Dickkopf-related protein 1 in a normal sample is indicative of preeclampsia.

Optionally, the method comprises the steps of:
(a) determining the quantitative level of Dickkopf-related protein 1 and/or platelet factor 4 in a sample from the subject;
(b) comparing the quantitative level of Dickkopf-related protein 1 and/or platelet factor 4 in the sample from the subject with the quantitative level of Dickkopf-related protein 1 and/or platelet factor 4 in a normal sample; and
(c) diagnosing preeclampsia in the subject based on the quantitative level of Dickkopf-related protein 1 and/or platelet factor 4;
wherein a quantitative level of Dickkopf-related protein 1 and/or platelet factor 4 in the sample from the subject greater than a quantitative level of Dickkopf-related protein 1 and/or platelet factor 4 in a normal sample is indicative of preeclampsia.

Optionally, the method comprises the steps of:
(a) determining the quantitative level of Dickkopf-related protein 1 in a sample from the subject;
(b) comparing the quantitative level of Dickkopf-related protein 1 in the sample from the subject with the quantitative level of Dickkopf-related protein 1 in a normal sample; and
(c) diagnosing preeclampsia in the subject based on the quantitative level of Dickkopf-related protein 1;
wherein a quantitative level of Dickkopf-related protein 1 in the sample from the subject greater than a quantitative level of Dickkopf-related protein 1 in a normal sample is indicative of term preeclampsia.

Optionally, determining comprises determining the quantitative level of a first set of biomarkers and the quantitative level of a second set of biomarkers.

Optionally, determining comprises determining the quantitative level of a first set of biomarkers relative to the quantitative level of a second set of biomarkers.

Optionally, the first set of biomarker is selected from Dickkopf-related protein 1 (Dkk1) and platelet factor 4 (PF4).

Optionally, the second set of biomarker is placental growth factor (PIGF).

Optionally, determining comprises determining the quantitative level of platelet factor 4 relative to the quantitative level of placental growth factor.

Optionally, determining comprises determining the quantitative level of Dickkopf-related protein 1 relative to the quantitative level of placental growth factor.

Optionally, the diagnosing step (b) comprises comparing the quantitative level of a first set of biomarkers relative to the quantitative level of a second set of biomarkers in the sample from the subject with the quantitative level of a first set of biomarkers relative to the quantitative level of a second set of biomarkers in a normal sample.

Optionally, the normal sample is a sample from a subject not suffering from preeclampsia.

Optionally, the normal sample is a sample from a healthy subject.

Optionally, a quantitative level of a first set of biomarkers relative to the quantitative level of a second set of biomarkers in the sample from the subject greater than a quantitative level of a first set of biomarkers relative to the quantitative level of a second set of biomarkers in a normal sample is indicative of preeclampsia.

Optionally, a quantitative level of Dickkopf-related protein 1 relative to the quantitative level of placental growth factor in the sample from the subject greater than a quantitative level of Dickkopf-related protein 1 relative to the quantitative level of placental growth factor in a normal sample is indicative of preeclampsia.

Optionally, a quantitative level of platelet factor 4 relative to the quantitative level of placental growth factor in the sample from the subject greater than a quantitative level of platelet factor 4 relative to the quantitative level of placental growth factor in a normal sample is indicative of preeclampsia.

Optionally, the method comprises the steps of:
(a) determining the quantitative level of Dickkopf-related protein 1 relative to the quantitative level of placental growth factor in a sample from the subject;
(b) comparing the quantitative level of determining the quantitative level of Dickkopf-related protein 1 relative to the quantitative level of placental growth factor in the sample from the subject with the quantitative level of determining the quantitative level of Dickkopf-related protein 1 relative to the quantitative level of placental growth factor in a normal sample; and
(c) diagnosing preeclampsia in the subject based on the quantitative level of determining the quantitative level of Dickkopf-related protein 1 relative to the quantitative level of placental growth factor;
wherein a quantitative level of Dickkopf-related protein 1 relative to the quantitative level of placental growth factor in the sample from the subject greater than a quantitative level of Dickkopf-related protein 1 relative to the quantitative level of placental growth factor in a normal sample is indicative of preeclampsia.

Optionally, the method comprises the steps of:
(a) determining the quantitative level of platelet factor 4 relative to the quantitative level of placental growth factor in a sample from the subject;
(b) comparing the quantitative level of determining the quantitative level of platelet factor 4 relative to the quantitative level of placental growth factor in the sample from the subject with the quantitative level of determining the quantitative level of platelet factor 4 relative to the quantitative level of placental growth factor in a normal sample; and
(c) diagnosing preeclampsia in the subject based on the quantitative level of determining the quantitative level of platelet factor 4 relative to the quantitative level of placental growth factor;
wherein a quantitative level of platelet factor 4 relative to the quantitative level of placental growth factor in the sample from the subject greater than a quantitative level of platelet factor 4 relative to the quantitative level of placental growth factor in a normal sample is indicative of preeclampsia.

According to a second aspect of the present invention, there is provided a method of diagnosing preeclampsia in a subject, the method comprising the steps of:
(a) determining the quantitative level of one or more biomarkers in a sample from the subject; and
(b) diagnosing preeclampsia in the subject based on the quantitative level of the or each biomarker in the sample;
wherein the or each biomarker is selected from CD41, CD42b, and CD62P.

Optionally, the biomarker is CD41, CD42b, and CD62P.

Optionally, the biomarker is CD41 and CD42b.

Optionally, the biomarker is CD41.

Optionally, the biomarker is CD41 and is not CD42b or CD62P.

Optionally, the biomarker is a particle expressing CD41, CD42b, and/or CD62P.

Optionally, the biomarker is a particle expressing CD41 and CD42b.

Optionally, the biomarker is a particle expressing CD41.

Optionally, the biomarker is a particle expressing CD41 and not expressing CD42b or CD62P.

Optionally, the particle is a vesicle.

Optionally, the particle is an extracellular vesicle.

Optionally, the biomarker is an extracellular vesicle expressing CD41, CD42b, and/or CD62P.

Optionally, the biomarker is an extracellular vesicle expressing CD41 and CD42b.

Optionally, the biomarker is an extracellular vesicle expressing CD41.

Optionally, the biomarker is an extracellular vesicle expressing CD41 and not expressing CD42b or CD62P.

Optionally, the diagnosing step (b) comprises comparing the quantitative level of the or each biomarker in the sample from the subject with the quantitative level of the or each respective biomarkers in a normal sample.

Optionally, the normal sample is a sample from a subject not suffering from preeclampsia.

Optionally, the normal sample is a sample from a healthy subject.

Optionally, a quantitative level of the or each biomarker in the sample from the subject greater than a quantitative level of the or each respective biomarkers in a normal sample is indicative of preeclampsia.

Optionally, a quantitative level of CD41, CD42b, and CD62P in the sample from the subject greater than a quantitative level of CD41, CD42b, and CD62P in a normal sample is indicative of preeclampsia.

Optionally, a quantitative level of CD41 and CD42b in the sample from the subject greater than a quantitative level of CD41 and CD42b in a normal sample is indicative of preeclampsia.

Optionally, a quantitative level of CD41 in the sample from the subject greater than a quantitative level of CD41 in a normal sample is indicative of preeclampsia.

Optionally, a quantitative level of an extracellular vesicle expressing CD41, CD42b, and/or CD62P in the sample from the subject greater than a quantitative level of an extracellular vesicle expressing CD41, CD42b, and/or CD62P in a normal sample is indicative of preeclampsia.

Optionally, a quantitative level of an extracellular vesicle expressing CD41 and CD42b in the sample from the subject greater than a quantitative level of an extracellular vesicle expressing CD41 and CD42b in a normal sample is indicative of preeclampsia.

Optionally, a quantitative level of an extracellular vesicle expressing CD41 in the sample from the subject greater than a quantitative level of an extracellular vesicle expressing CD41 in a normal sample is indicative of preeclampsia.

Optionally, a quantitative level of an extracellular vesicle expressing CD41 and not expressing CD42b or CD62P in the sample from the subject greater than a quantitative level of an extracellular vesicle expressing CD41 and not expressing CD42b or CD62P in a normal sample is indicative of preeclampsia.

Optionally, a quantitative level of an extracellular vesicle expressing CD41 and not expressing CD42b or CD62P in the sample from the subject greater than a quantitative level of an extracellular vesicle expressing CD41 and not expressing CD42b or CD62P in a normal sample is indicative of term preeclampsia.

Optionally, the method comprises the steps of:
(a) determining the quantitative level of an extracellular vesicle expressing CD41, CD42b, and/or CD62P in a sample from the subject;
(b) comparing the quantitative level of the extracellular vesicle expressing CD41, CD42b, and/or CD62P in the sample from the subject with the quantitative level of the extracellular vesicle expressing CD41, CD42b, and/or CD62P in a normal sample and
(c) diagnosing preeclampsia in the subject based on the quantitative level of extracellular vesicle expressing CD41, CD42b, and/or CD62P;
wherein a quantitative level of an extracellular vesicle expressing CD41, CD42b, and/or CD62P in the sample from the subject greater than a quantitative level of an extracellular vesicle expressing CD41, CD42b, and/or CD62P in a normal sample is indicative of preeclampsia.

Optionally, the method comprises the steps of:
(a) determining the quantitative level of an extracellular vesicle expressing CD41, CD42b, and/or CD62P in a sample from the subject;
(b) comparing the quantitative level of the extracellular vesicle expressing CD41, CD42b, and/or CD62P in the sample from the subject with the quantitative level of the extracellular vesicle expressing CD41, CD42b, and/or CD62P in a normal sample and
(c) diagnosing preeclampsia in the subject based on the quantitative level of extracellular vesicle expressing CD41, CD42b, and/or CD62P;
wherein a quantitative level of an extracellular vesicle expressing CD41 and not expressing CD42b or CD62P in the sample from the subject greater than a quantitative level of an extracellular vesicle expressing CD41 and not expressing CD42b or CD62P in a normal sample is indicative of term preeclampsia.

According to a third aspect of the present invention, there is provided a method of diagnosing preeclampsia in a subject, the method comprising the steps of:
(a) determining the quantitative level of one or more biomarkers in a sample from the subject; and
(b) diagnosing preeclampsia in the subject based on the quantitative level of the or each biomarker in the sample;
wherein the biomarker is a particle having a diameter of 100 - 1100nm.

Optionally, the particle has an average diameter of 100 - 1100.

Optionally, the particle has a diameter of 100 - 1100, optionally 200 - 1000, optionally 300 - 900, optionally 400 - 800, optionally 500 - 700, optionally 500 - 600nm.

Optionally, the particle has a diameter of 100 - 200, 200 - 300, 300 - 400, 400 - 500, 500 - 600, 600 - 700, 700 - 800, 800 - 900, 900 - 1000, or 1000 - 1100nm.

Preferably, the particle has a diameter of 500 - 600nm.

Optionally, the particle has a diameter of 101 - 200, 201 - 300, 301 - 400, 401 - 500, 501 - 600, 601 - 700, 701 - 800, 801 - 900, 901 - 1000, or 1001 - 1100nm.

Preferably, the particle has a diameter of 501 - 600nm.

Optionally, the diagnosing step (b) comprises comparing the quantitative level of the or each biomarker in the sample from the subject with the quantitative level of the or each respective biomarkers in a normal sample.

Optionally, the normal sample is a sample from a subject not suffering from preeclampsia.

Optionally, the normal sample is a sample from a healthy subject.

Optionally, a quantitative level of the or each biomarker in the sample from the subject greater than a quantitative level of the or each respective biomarkers in a normal sample is indicative of preeclampsia.

Optionally, a quantitative level of a particle having a diameter of 100 - 1100nm in the sample from the subject greater than a quantitative level of a particle having a diameter of 100 - 1100nm in a normal sample is indicative of preeclampsia.

Optionally, a quantitative level of a particle having a diameter of 500 - 600nm in the sample from the subject greater than a quantitative level of a particle having a diameter of 500 - 600nm in a normal sample is indicative of preeclampsia.

Optionally, the particle is a vesicle.

Optionally, the particle is an extracellular vesicle.

Optionally, a quantitative level of an extracellular vesicle having a diameter of 500 - 600nm in the sample from the subject greater than a quantitative level of an extracellular vesicle having a diameter of 500 - 600nm in a normal sample is indicative of preeclampsia.

Optionally, the method comprises the steps of:
(a) determining the quantitative level of an extracellular vesicle having a diameter of 500 - 600nm in a sample from the subject;
(b) comparing the quantitative level of an extracellular vesicle having a diameter of 500 - 600nm in the sample from the subject with the quantitative level of an extracellular vesicle having a diameter of 500 - 600nm in a normal sample and
(c) diagnosing preeclampsia in the subject based on the quantitative level of the extracellular vesicle having a diameter of 500 - 600nm;
wherein a quantitative level of an extracellular vesicle having a diameter of 500 - 600nm in the sample from the subject greater than a quantitative level of an extracellular vesicle having a diameter of 500 - 600nm in a normal sample is indicative of preeclampsia.

Optionally, the method is a method of diagnosing pre-term preeclampsia in a subject.

Optionally, pre-term preeclampsia is preeclampsia suffered by a pregnant subject of less than 37 weeks gestation.

Optionally, the method is a method of diagnosing term preeclampsia in a subject.

Optionally, term preeclampsia is preeclampsia suffered by a pregnant subject of equal to or more than 37 weeks gestation.

Optionally, the subject is a human.

Optionally, the subject is a female.

Optionally, the subject is a female human.

Preferably, the subject is a female human.

Optionally, the subject is a pregnant subject.

Preferably, the subject is a pregnant subject.

Optionally, the subject is a pregnant subject of less than 28 weeks gestation.

Optionally, the subject is a pregnant subject of less than 28, optionally lees than 24, optionally lees than 20, optionally lees than 16, optionally lees than 12, optionally lees than 8, optionally lees than 4 weeks gestation.

Optionally, the subject is a pregnant subject of 28 - 4, optionally 24 - 8, optionally 20 - 12, optionally 16 - 12 weeks gestation.

Optionally, the sample is selected from platelet releasates, whole blood, serum, plasma, urine, interstitial fluid, peritoneal fluid, cervical swab, tears, saliva, buccal swab, skin, brain tissue, and cerebrospinal fluid.

Optionally, the sample is a plasma sample.

Optionally, the sample is a plasma sample comprising an anticoagulant.

Optionally, the sample is a plasma sample contacted by an anticoagulant.

Optionally, the anticoagulant is selected from EDTA, citrate, and heparin.

Preferably, the anticoagulant is EDTA.

Optionally, the sample is a platelet-poor plasma (PPP) sample.

Optionally, the sample is a plasma sample having less than 10x10e3 platelets per µL of plasma sample.

Optionally, the method further comprises the step of treating the subject in need thereof.

Optionally, treating the subject in need thereof comprises delivering the baby.

Optionally, treating the subject in need thereof comprises delivering the baby up to 37 weeks gestation.

Alternatively, treating the subject in need thereof comprises delivering the baby after 37 weeks gestation.

Optionally, treating the subject in need thereof comprises administering an effective amount of an agent suitable for treating hypertension.

Optionally, treating the subject in need thereof comprises administering an effective amount of an agent suitable for treating pregnancy-induced or gestational hypertension.

Optionally, treating the subject in need thereof comprises administering an effective amount of aspirin, labetalol ((RS)-2-Hydroxy-5-[1-hydroxy-2-[(4-phenylbutan-2-yl)amino]ethyl]benzamide), methyldopa ((S)-2-amino-3-(3,4-dihydroxyphenyl)-2-methyl-propanoic acid), and magnesium sulfate.

Optionally, treating is conducted before 28 weeks gestation.

Optionally, treating is conducted before 28, optionally before 24, optionally before 20, optionally before 16, optionally before 12, optionally before 8, optionally before 4 weeks gestation.

Optionally, treating is conducted at 28 - 4, optionally 24 - 8, optionally 20 - 12, optionally 16-12 weeks gestation.

Optionally, the method is a method of treating preeclampsia in a subject.

Optionally, the or each biomarker is a gene.

Optionally, the or each biomarker is a nucleic acid.

Optionally, the or each biomarker is a deoxyribonucleic acid.

Optionally, the or each biomarker is a ribonucleic acid.

Optionally, the or each biomarker is a protein.

Optionally, the or each biomarker is a peptide.

Optionally, the biomarker is Dickkopf-related protein 1 (Dkk1).

Optionally, the biomarker is the Dkk1 protein.

Optionally, the biomarker is the Dkk1 protein having the UniProt ID 094907.

Optionally, the biomarker is the Dkk1 protein having the NCBI Reference Sequence Accession/Version No NP_036374.1.

Optionally, the biomarker is the DKK1 nucleic acid encoding the Dkk1 protein.

Optionally, the biomarker is the DKK1 nucleic acid having the Gene ID 22943.

Optionally, the biomarker is the DKK1 nucleic acid having the NCBI Reference Sequence Accession/Version No NM_012242.4.

Optionally, the biomarker is platelet factor 4 (PF4).

Optionally, the biomarker is the PF4 protein.

Optionally, the biomarker is the PF4 protein having the UniProt ID P02776.

Optionally, the biomarker is the PF4 protein having the NCBI Reference Sequence Accession/Version No NP_001350281.1.

Optionally, the biomarker is the PF4 nucleic acid encoding the PF4 protein.

Optionally, the biomarker is the PF4 nucleic acid having the Gene ID 5196.

Optionally, the biomarker is the PF4 nucleic acid having the NCBI Reference Sequence Accession/Version No NM_001363352.1.

Optionally, the biomarker is CD41 (Integrin alpha-IIb).

Optionally, the biomarker is the CD41 (Integrin alpha-IIb) protein.

Optionally, the biomarker is the CD41 (Integrin alpha-IIb) protein having the UniProt ID P08514.

Optionally, the biomarker is the CD41 (Integrin alpha-IIb) protein having the NCBI Reference Sequence Accession/Version No NP_000410.2.

Optionally, the biomarker is the ITGA2B nucleic acid encoding the CD41 (Integrin alpha-IIb) protein.

Optionally, the biomarker is the ITGA2B nucleic acid having the Gene ID 3674.

Optionally, the biomarker is the ITGA2B nucleic acid having the NCBI Reference Sequence Accession/Version No NM_000419.5.

Optionally, the biomarker is CD42b (glycoprotein Ib platelet subunit alpha).

Optionally, the biomarker is the CD42b (glycoprotein Ib platelet subunit alpha) protein.

Optionally, the biomarker is the CD42b (glycoprotein Ib platelet subunit alpha) protein having the UniProt ID P07359.

Optionally, the biomarker is the CD42b (glycoprotein Ib platelet subunit alpha) protein having the NCBI Reference Sequence Accession/Version No NP_000164.5.

Optionally, the biomarker is the GP1BA nucleic acid encoding the CD42b (glycoprotein Ib platelet subunit alpha) protein.

Optionally, the biomarker is the GP1BA nucleic acid having the Gene ID 2811.

Optionally, the biomarker is the GP1BA nucleic acid having the NCBI Reference Sequence Accession/Version No NM_000173.7.

Optionally, the biomarker is CD62P (P-selectin).

Optionally, the biomarker is the CD62P (P-selectin) protein.

Optionally, the biomarker is the CD62P (P-selectin) protein having the UniProt ID P16109.

Optionally, the biomarker is the CD62P (P-selectin) protein having the NCBI Reference Sequence Accession/Version No NP_002996.2.

Optionally, the biomarker is the SELPB nucleic acid encoding the CD42b (glycoprotein Ib platelet subunit alpha) protein.

Optionally, the biomarker is the SELP nucleic acid having the Gene ID 6403.

Optionally, the biomarker is the SELP nucleic acid having the NCBI Reference Sequence Accession/Version No NM_003005.4.

Optionally, the biomarker is placental growth factor (PIGF).

Optionally, the biomarker is the PIGF protein.

Optionally, the biomarker is the PIGF protein having the UniProt ID P49763-1.

Optionally, the biomarker is the PIGF protein having the NCBI Reference Sequence Accession/Version No NP_001193941.1.

Optionally, the biomarker is the PGF nucleic acid encoding the PIGF protein.

Optionally, the biomarker is the PGF nucleic acid having the Gene ID 5228.

Optionally, the biomarker is the PGF nucleic acid having the NCBI Reference Sequence Accession/Version No NM_001207012.1.

Optionally, the determining step (a) comprises determining the quantitative level of all of the biomarkers in the sample from the subject.

Optionally or additionally, the determining step (a) comprises determining the quantitative level of each of the biomarkers in the sample from the subject.

Optionally, the method of diagnosing preeclampsia is an in vitro method.

Optionally, the method is a method of screening for preeclampsia in a subject, the method comprising the steps of:
(a) determining the quantitative level of one or more biomarkers in a sample from the subject; and
(b) screening for preeclampsia in the subject based on the quantitative level of the or each biomarker in the sample.

Optionally, a quantitative level of the or each biomarker in the sample from the subject greater than a quantitative level of the or each respective biomarkers in a normal sample is indicative of preeclampsia.

Optionally, the method is a method of determining the likelihood that a subject will suffer from preeclampsia, the method comprising the steps of:
(a) determining the quantitative level of one or more biomarkers in a sample from the subject; and
(b) determining the likelihood that a subject will suffer from preeclampsia based on the quantitative level of the or each biomarker in the sample.

Optionally, a quantitative level of the or each biomarker in the sample from the subject greater than a quantitative level of the or each respective biomarkers in a normal sample is indicative that a subject will suffer from preeclampsia.

Optionally, the method is a method of determining the risk that a subject will suffer from preeclampsia, the method comprising the steps of:
(a) determining the quantitative level of one or more biomarkers in a sample from the subject; and
(b) determining the risk that a subject will suffer from preeclampsia based on the quantitative level of the or each biomarker in the sample.

Optionally, a quantitative level of the or each biomarker in the sample from the subject greater than a quantitative level of the or each respective biomarkers in a normal sample is indicative that a subject is at risk of suffering from preeclampsia.

### Brief description of the drawings

Embodiments of the present invention will now be described with reference to the following nonlimiting examples and the accompanying drawings, in which:
**Figure 1** illustrates flow cytometric analysis of circulating particles in plasma during early pregnancy, revealing a significant increase in particle counts in pregnancies later complicated by either preterm PE (n=41) or term PE (n=51), compared to control pregnancies (n=244);
**Figure 2** illustrates that patients that later develop preterm (n=40) or term (n=50) PE in their pregnancies have a significant increase in platelet/megakaryocyte lineage derived extracellular vesicles (EVs) compared to control (n=131) from 11 weeks' gestation;
**Figure 3** illustrates subpopulations of platelet/megakaryocyte lineage derived EVs that are significantly altered between preterm, term and control;
**Figure 4** illustrates PF4 and Dkk1 are significantly increased from 11 weeks' gestation in those that subsequently develop PE in their pregnancies;
**Figure 5** illustrates PF4 and Dkk1 as a ratio to PIGF separately display a significant increased ratio in those that develop preterm PE compared to control;

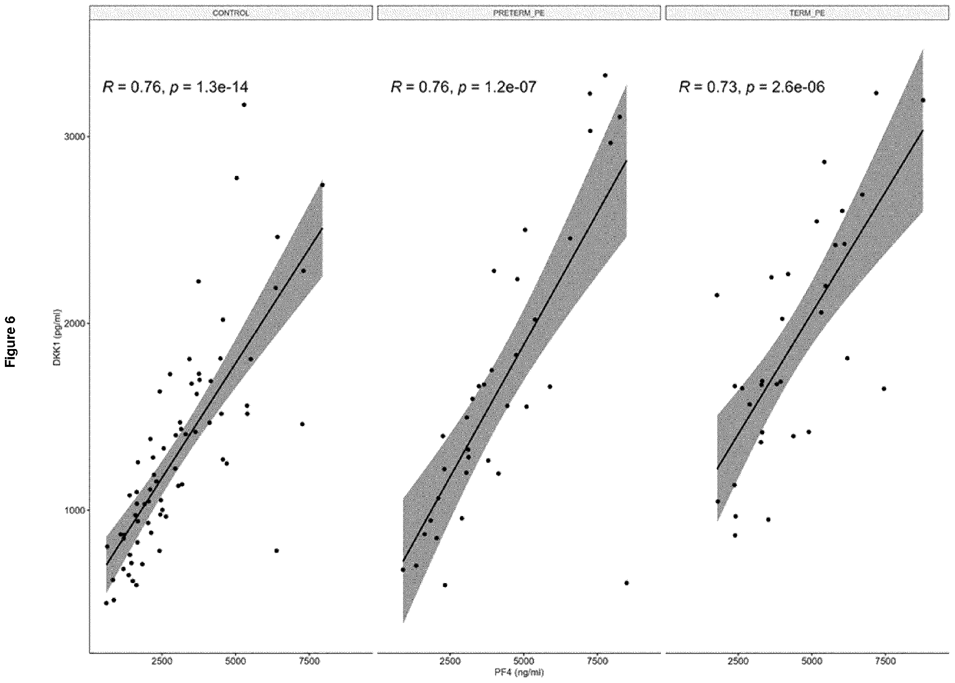

**Figure 6** illustrates PF4 and Dkk1 are significantly positively correlated (R=0.76, R=0.76 and R=0.73) in the control (n=103), PRETERM_PE (n=41) and TERM_PE (n=49) groups respectively; and
**Figure 7** illustrates a gating strategy for multicolour flow cytometry.

### Examples

### Materials and methods

### Patient recruitment

Ethics were granted (LS-E-21-242-Maguire) to gain access to EDTA plasma samples from the Biological Resource bank in the Coombe Women and Infants University Hospital Dublin in October 2021. Plasma samples taken from women in early pregnancy (11-16 weeks gestation) were obtained for those that later had a pregnancy complicated by preterm (n=41) or term (n=51) pre-eclampsia (PE) and compared in a case-control manner to gestationally-aged-matched women that did not develop PE in their pregnancies (n=244). Preterm PE was defined as a clinical diagnosis of PE and delivery <37 weeks' gestation, and term PE defined as a clinical diagnosis of PE and delivery >37 weeks' gestation. Plasma samples were stored at -80°C. Platelet Poor Plasma (PPP) samples were further clarified to ensure removal of cell debris by centrifugation at 3000g at 4°C for 10 minutes, aliquoted and stored at -80°C until use.

### Extracellular particle quantification

Flow cytometric analysis of circulating extracellular particles in plasma samples was performed using a CytoFlex S (Beckman Coulter, Brea, CA, USA). 30µL PPP was diluted with 520µl 0.22µm-filtered PBS. Samples were further diluted 1:50 to prevent EV swarming and assayed in triplicate at a constant flow rate of 1µL/min for 150 seconds or until 100,000 events were recorded. A buffer-only sample (0.22µm filtered PBS) was assayed in between patient samples using the same settings to ensure sufficiently low background between samples. To quantify particle size and account for differences in refractive indices between reference beads and biological particles, Rosetta calibration beads (Exometry, Amsterdam, the Netherlands) were run daily and associated calibration software (V2_06) was used. Sample calculation parameters assumed a core/shell, medium refractive index of 1.3431, particle core refractive index of 1.37, particles shell/core refractive index of 1.45, and particle thickness of 4nm. Samples were assayed in triplicate and average of replicates used for analysis. Output .fcs files were analysed using Kaluza analysis software (Beckman Coulter v_2.2) and particles were quantified in 100nm bins from 200-1000nm.

### Identification of extracellular vesicle cell of origin

Multicolour flow cytometry was used for phenotyping of circulating large EVs in plasma samples. EVs were enriched from 100µL PPP by centrifugation at 20,800xg at 10°C for 10 minutes in an Eppendorf 5810R centrifuge with an SL-140 rotor. EV pellets were resuspended in 1mL filtered PBS, vortexed and spun again. Washed EV pellets were resuspended in 230µL Annexin V Binding Buffer (BD Pharmingen). The subcellular origin was determined by flow cytometry (CytoFlex S, Beckman Coulter, Brea, CA, USA) using cell-type-specific surface antigens for platelet/megakaryocyte lineage (CD41- pacific blue (#303714, BioLegend), CD42b-APC (#303912, BioLegend), and CD62P-PE (#304906, BioLegend)). Large EVs were identified based on size and Annexin V-FITC (#640945, BioLegend) labelling. Rosetta beads and associated software were used to make size gate cut offs.

### Enzyme-linked immunosorbent assay (ELISA)

Quantification of biomarkers was carried out using Quantakine ELISA kits from R and D Systems (R&D Systems, Minneapolis, MN, USA). For quantification of Platelet Factor 4 (PF4), PPP was diluted 1:200 and assayed according to the manufacturer's instructions (#DPF40, R&D Systems). For quantification of Dkk1, PPP was diluted 1:10 and assayed according to the manufacturer's instructions (DKK100B, R&D Systems). All standards and samples were assayed in technical duplicate.

### Data analysis

Data analysis was performed in R Studio. Q-Q plots and Shapiro-Wilk test confirmed non-normal distribution of data. Group comparisons were conducted using the Kruskal-Wallis rank-sum test, with pairwise p values obtained via Dunn's test and adjusted for multiple comparisons using the Benjamini-Hochberg (BH) correction. Where appropriate further adjustment of p values was conducted for multiple testing.

### Example 1

### Extracellular particle quantification

Quantification of circulating particles between 200-1000nm in plasma using flow cytometry revealed a significant increase in total particle count in individuals who subsequently developed PE. In particular, particles 500-600nm in size showed the most pronounced elevation in PE groups (both preterm and term) when compared to the control group. This early-stage elevation in circulating particles, observed before symptom onset, provides evidence of altered particle profiles that may contribute to the disease's pathophysiology.

### Example 2

### Identification of extracellular vesicle cell of origin

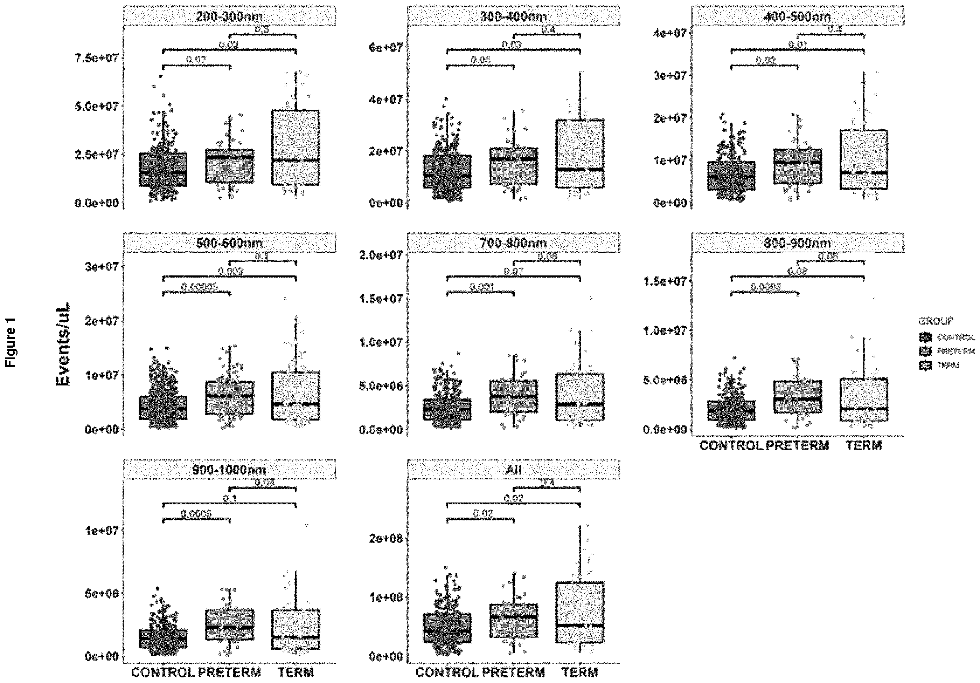

To investigate the cellular origin of the increase in circulating particles, EVs were further probed using multicolour flow cytometry for platelet/megakaryocyte lineage markers of origin - CD41, CD42b and CD62P. Vesicles were gated for positive phosphatidylserine staining and co-expression of markers listed. A significant increase in all markers was observed in both preterm and term groups compared to control (see Figure 1).

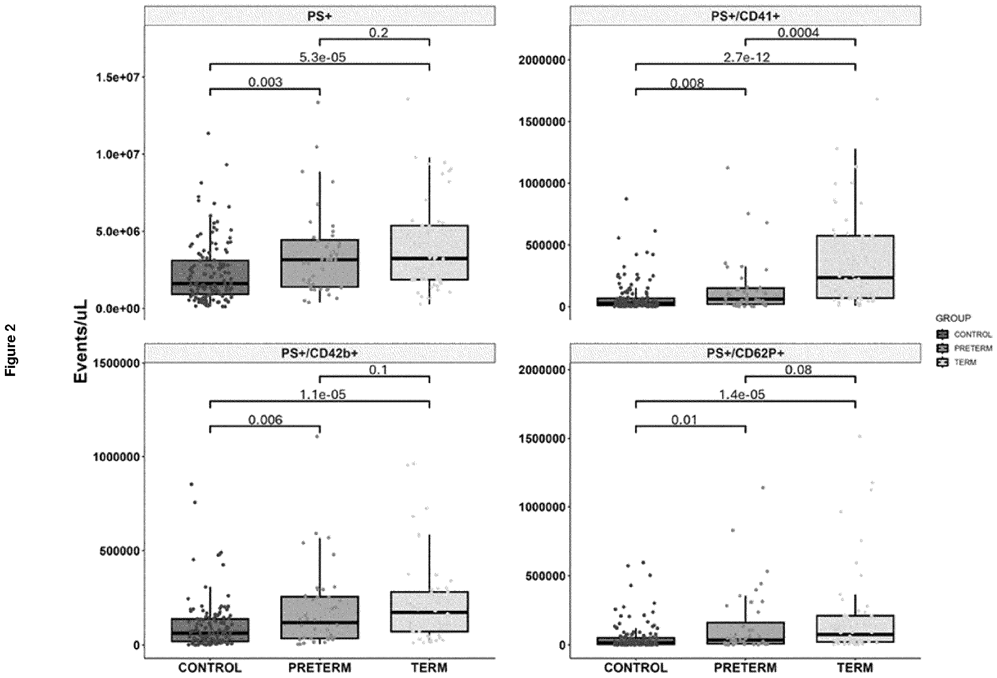

Figure 2 shows vesicles were gated for positive phosphatidylserine staining. Significant increases in platelet/megakaryocyte EVs positive for PS+/CD41 + and PS+/CD42b+ and EVs from activated platelets PS+/CD41 + were observed in PE groups compared to control.

Further analysis of distinct subpopulations of platelet/megakaryocyte lineage EVs uncovered more unique insights into specific differences between the two subgroups of PE. Megakaryocyte derived EVs defined in this case as PS+/CD41+/CD42b-/CD62P- are significantly altered in the term PE group compared to preterm PE and control groups. This unique observation of a distinct difference between preterm and term groups provides evidence of distinct phenotypes for preterm and term PE and is evidenced in this study population from 11 weeks' gestation. Common to both subtypes of PE and most significantly increased from control, are the procoagulant platelet PS+/CD41+/CD42b+/CD62P+ EVs. These EVs exposing CD62P on their surface are significantly increased from 11 weeks of gestation in those that subsequently develop PE (see Figure 2).

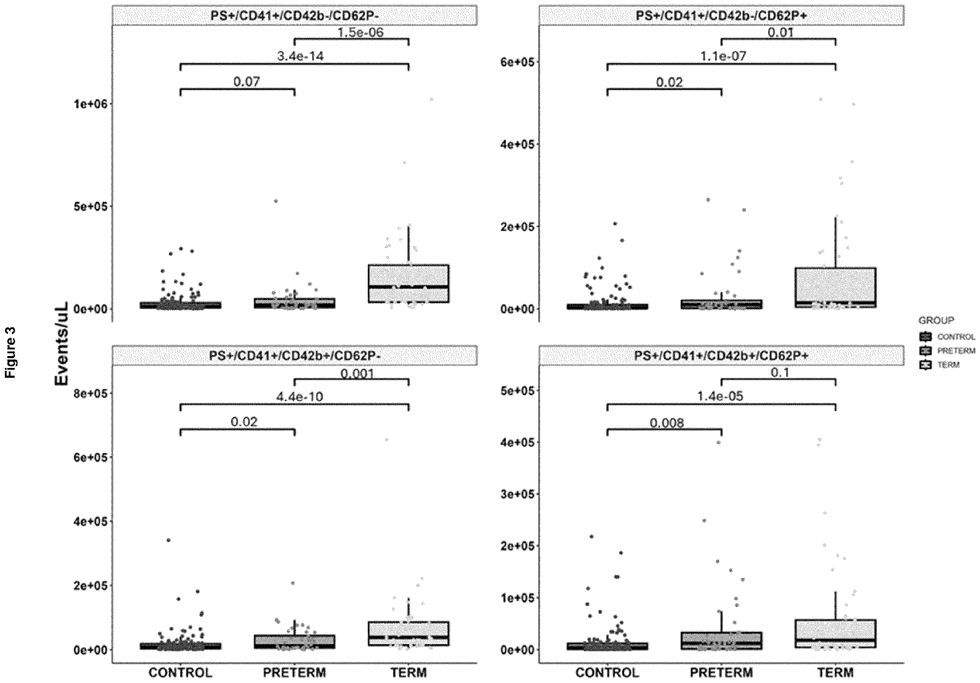

Figure 3 shows term PE (n=50) has a significant elevation in megakaryocyte derived EVs (PS+/CD41+/CD42b-/CD62P-) comparted to preterm (n=40) and control (n=131) groups in early pregnancy. Most significant and unique to PE groups was a significant increase in pro-coagulant platelet derived EVs (PS+/CD41+/CD42b+/CD62P+) compared to control.

### Example 3

### Quantification of biomarkers

The differences revealed by flow cytometric analysis of circulating EVs showed a significant increase in EVs derived from activated platelets (PS+/CD41+/CD42b+/CD62P+) (see Figure 3). Following platelet activation, there is release of alpha granule proteins from the platelet. Using ELISA, levels of PF4 and Dkk1 were measured in plasma and a significant increase in both PF4 and Dkk1 from 11 weeks' gestation in the plasma of women that later developed preterm or term PE in their pregnancies was observed. PF4 plays an important role in thrombosis and haemostasis and acts as an inhibitor of megakaryocytopoiesis and angiogenesis. Although platelet-derived factors aid in the differentiation of cells in the growing placenta, a significant increase in anti-angiogenic PF4 in maternal plasma in early pregnancy may be detrimental to a growing placenta when growth and invasion of trophoblast cells are paramount. Dkk1, which is also stored in platelet alpha granules and released following activation, is an antagonist of the canonical Wnt signalling pathway and an increase in soluble Dkk1 may inhibit the proliferation and invasion of placental cells. Like PF4, a significant increase in Dkk1 may be pathological and contributing to the placental insufficiently and dysfunction associated with PE.

The use of these biomarkers was investigated as a ratio with PIGF, a protein variable useful in prediction models for PE. Due to limited plasma this was initially assayed in the preterm PE group only.

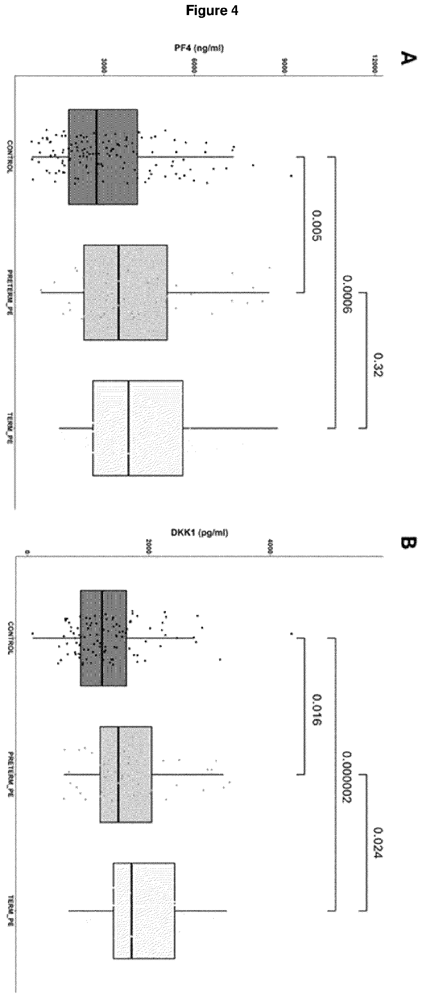

Figure 4(A) shows levels of PF4, as measured by ELISA, showed no statistical significance between preterm preeclampsia (PRETERM_PE, n=43) and term preeclampsia (TERM_PE, n=54). Both PE subgroups separately demonstrated a statistically significant increase in PF4 when compared to control (n=146) pregnancies (p= 0.005 and p= 0.0006 respectively). Figure 4(B) shows levels of Dkk1, as measured by ELISA, showed statistical significance between preterm preeclampsia (PRETERM_PE, n=41) and term preeclampsia (TERM_PE, n=49), (p=0.024). Both preeclampsia subgroups separately demonstrated a statistically significant increase in Dkk1 when compared to control (n=103) pregnancies (p= 0.016 and p= 0.000002 respectively). Women were between 11-15 weeks of gestation at time of blood sampling.

Both the PF4:PIGF and Dkk1:PIGF ratio were significantly increased from 11 weeks of gestation in those that went on to develop preterm PE compared to control and demonstrated that these markers separated out cases from controls better than the markers alone. The utility of these biomarkers together can improve the separation of groups, and a potential cut-off value used for risk stratification.

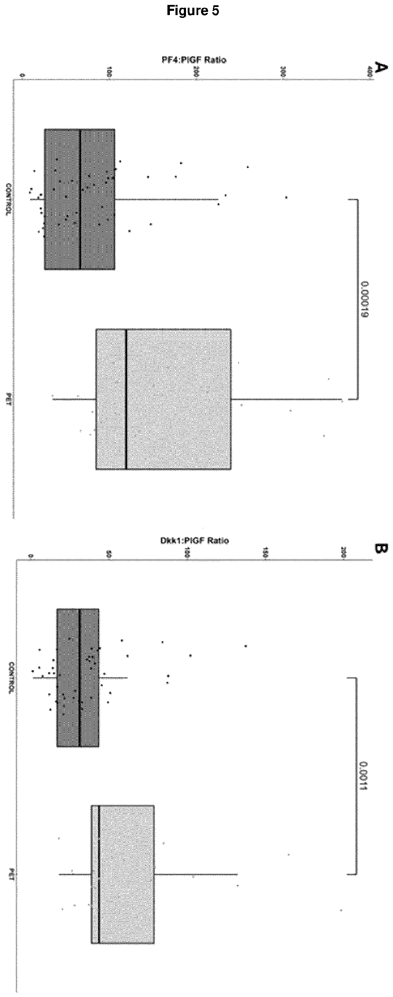

Figure 5(A) shows the PF4:PIGF ratio is significantly increased (p=0.00019) in women who were diagnosed after 20 weeks of gestation with preterm preeclampsia (PRETERM_PE, n=22) when compared to control (n=49). Figure 5(B) shows the Dkk1:PIGF ratio is significantly increased (p=0.0011) in women who were diagnosed after 20 weeks of gestation with preterm preeclampsia (PRETERM_PE, n=22) when compared to control (n=47). All women were between 11-15 weeks of gestation at time of blood sampling.

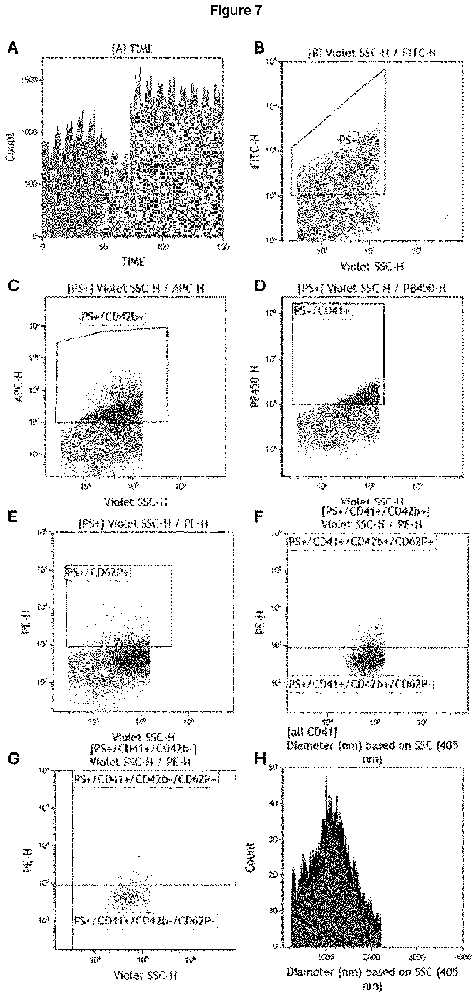

Figure 7(A) shows events are gated for a stable time of 100 seconds. Figure 7(B) shows PS positive events are gated based on size and positivity of FITC. Figure 7(C) shows double positive PS and CD42b gate. Figure 7(D) shows double positive PS and CD41 gate. Figure 7(E) shows double positive PS and CD62P gate. Figure 7(F) shows triple positive events as PS+/CD41+/CD42b+/CD62P+ (upper quadrant) and PS+/CD41+/CD42b+/CD62P- (lower quadrant). Figure 7(G) shows triple positive events as PS+/CD41+/CD42b-/CD62P+ (upper quadrant) and PS+/CD41+/CD42b-/CD62P- (lower quadrant). Figure 7(H) shows a representative size of CD41 gate to ensure correct upper and lower limits of EV sizes.

## Claims

1. A method of diagnosing preeclampsia in a subject, the method comprising the steps of:
(a) determining the quantitative level of one or more biomarkers in a sample from the subject; and
(b) diagnosing preeclampsia in the subject based on the quantitative level of the or each biomarker in the sample;
wherein the or each biomarker is selected from Dickkopf-related protein 1 (Dkk1) and platelet factor 4 (PF4).

2. A method according to claim 1, wherein the subject is a pregnant subject of 16 - 12 weeks gestation.

3. A method according to claim 1 or 2, wherein diagnosing comprises comparing the quantitative level of the or each biomarker in the sample from the subject with the quantitative level of the or each respective biomarkers in a normal sample, wherein a quantitative level of the or each biomarker in the sample from the subject greater than a quantitative level of the or each respective biomarkers in a normal sample is indicative of preeclampsia.

4. A method according to claim 3, wherein a quantitative level of Dickkopf-related protein 1 in the sample from the subject greater than a quantitative level of Dickkopf-related protein 1 in a normal sample is indicative of preeclampsia.

5. A method according to claim 3, wherein a quantitative level of platelet factor 4 in the sample from the subject greater than a quantitative level of platelet factor 4 in a normal sample is indicative of preeclampsia.

6. A method according to any one of claims 1-3, wherein determining comprises determining the quantitative level of Dickkopf-related protein 1 relative to the quantitative level of placental growth factor.

7. A method according to claim 6, wherein a quantitative level of Dickkopf-related protein 1 relative to the quantitative level of placental growth factor in the sample from the subject greater than a quantitative level of Dickkopf-related protein 1 relative to the quantitative level of placental growth factor in a normal sample is indicative of preeclampsia.

8. A method according to any one of claims 1-3, wherein determining comprises determining the quantitative level of platelet factor 4 relative to the quantitative level of placental growth factor.

9. A method according to claim 8, wherein a quantitative level of platelet factor 4 relative to the quantitative level of placental growth factor in the sample from the subject greater than a quantitative level of platelet factor 4 relative to the quantitative level of placental growth factor in a normal sample is indicative of preeclampsia.

10. A method according to any one of claims 1-9, wherein the or each biomarker is further selected from CD41, CD42b, and CD62P.

11. A method according to claim 10, wherein a quantitative level of CD41, CD42b, and/or CD62P in the sample from the subject greater than a quantitative level of CD41, CD42b, and/or CD62P in a normal sample is indicative of preeclampsia.

12. A method according to any one of claims 1-10, wherein the biomarker is an extracellular vesicle expressing CD41, CD42b, and/or CD62P.

13. A method according to claim 12, wherein the extracellular vesicle is expressing CD41 and not expressing CD42b or CD62P.

14. A method according to claim 13, wherein a quantitative level of an extracellular vesicle expressing CD41 and not expressing CD42b or CD62P in the sample from the subject greater than a quantitative level of an extracellular vesicle expressing CD41 and not expressing CD42b or CD62P in a normal sample is indicative of preeclampsia.

15. A method according to any one of claims 1-14, wherein the biomarker is an extracellular vesicle having a diameter of 100 - 1100nm.
